# EUROPEAN PATENT APPLICATION

(11) **EP 3 211 028 A1**
(43) Date of publication of application: **30.08.2017**
(21) Application number: 15853157.4
(22) Date of filing: 01.10.2015
(51) Int. Cl.: C08J 9/26, B01J 20/24, B01J 20/30, G01N 30/88

(54) **METHOD FOR PRODUCING POLYSACCHARIDE MONOLITH STRUCTURE**

(30) Priority: 21.10.2014 JP 2014214242
(71) Applicant: JNC Corporation, Chiyoda-ku Tokyo 100-8105 (JP)
(72) Inventor: OKANIWA, Natuki, Yokohama-shi Kanagawa 236-8605 (JP); NAKAMA, Tsuyoshi, Yokohama-shi Kanagawa 236-8605 (JP); IWAMOTO, Eri, Yokohama-shi Kanagawa 236-8605 (JP); SASAKI, Shuji, Yokohama-shi Kanagawa 236-8605 (JP); IKEDA, Takuo, Yokohama-shi Kanagawa 236-8605 (JP)
(74) Representative: Stoner, Gerard Patrick
(86) International application number: PCT/JP2015/077917
(87) International publication number: WO 2016/063702

(57) **Abstract**

Provided is a manufacturing method for monolithic structure that is a porous body formed of polysaccharide being a naturally-occurring polymer, has continuous pores with an average pore diameter suitable for biomolecule separation, and allows formation into arbitrary shape. The polysaccharide monolithic structure is manufactured by a method including a first step of obtaining a polysaccharide solution by dissolving polysaccharide into a mixed solvent of a first component and a second component at temperature lower than a boiling point of the mixed solvent, and a second step of obtaining polysaccharide monolithic structure by cooling the polysaccharide solution, wherein the first component is a solvent selected from lactate, and the second component is a solvent selected from water, lower alcohol, and a combination thereof. The monolithic structure obtained is a porous body having continuous pores with an average pore diameter of 0.01 to 20.0 micrometers, and a thickness of 100 micrometers or more.

## Description

### Technical Field

The invention relates to a manufacturing method for a polysaccharide monolithic structure.

### Background Art

Attention is focused on research and development of a biomedicine manufactured by using various animals and insects, and cells thereof and microorganisms each as a host. Productivity of the biomedicine is improving by high-capacity culture and high-titer culture, and achievement of high efficiency is required also for a purification step as associated therewith. The biomedicine is of biological origin, and therefore a very sophisticated purification technology is needed, and purification is made in combination of centrifugal separation, ultrafiltration, microfiltration, chromatography and so forth. In particular, the chromatography is a core technology of purification.

As media for chromatography used for separating protein, nucleic acid, a virus or the like to be used as a biomedicine raw material, porous particles having various pore diameters or porous particles into which a functional group is introduced in order to impart adsorption performance are manufactured and commercially available. A naturally-occurring polymer such as cellulose is used for most of the media for chromatography.

However, most of commercial items are available mainly for the purpose of separating protein having a molecular weight of about several hundreds of thousands, and only a limited item is suitable for a giant biopolymer such as nucleic acid having a molecular weight of one million or more. Such lack of a suitable item is caused by difficulty in increasing a pore diameter without adversely affecting pressure-resistant strength of particles.

As a porous material, attention has been recently drawn to a monolith being a single block of porous body continuously having three-dimensional network structure skeletons and voids, respectively, as a next-generation porous material. In the monolith, sizes of the skeletons and the pores to serve as channels can be independently controlled.

For example, a silica monolith formed using silica as a material is commercially available as a monolith column, and has high separability and excellent mechanical strength. Moreover, technology development is progressing for controlling a skeleton diameter and a channel pore and controlling surface micropores.

However, most of the technology development is directed to a capillary-sized silica monolith column having an inner diameter of 0.3 mm or less due to ease of preparation of a capillary-sized monolith column. More specifically, such a silica monolith is intended for analysis and does not have adsorption capacity that can be used for the purification step in biomedicine production.

In addition to silica, various reports have been made so far on a monolithic structure formed of a polymer material and a manufacturing method therefor (Patent literature Nos. 1 to 3).

Incidentally, a polysaccharide being a naturally-occurring polymer and a derivative thereof also have a large number of hydroxyl groups and are expected to have low interaction with a biological substance such as protein, and therefore the polysaccharide is utilized as a material of a porous membrane, and manufacturing methods therefor are disclosed (Patent literature Nos. 4 to 6). Patent literature No. 6, in particular, discloses a heat-induced phase separation method using a high boiling point solvent, as a manufacturing method for a porous membrane using a cellulose derivative.

### Citation List

### Patent Literature

Patent literature No. 1: JP 2009-030017 A.
Patent literature No. 2: JP 2012-107216 A.
Patent literature No. 3: JP 2010-260952 A.
Patent literature No. 4: JP 2006-082006 A.
Patent literature No. 5: WO 2013/118859 A.
Patent literature No. 6: JP 2003-001074 A.

### Summary of Invention

### Technical Problem

A material obtained according to the method described in Patent literature No. 6 is porous, in which pores thereof are not continuous pores but are independent pores, individually, and such a material is hard to be utilized as a column material. Moreover, the material has a film-shaped form having a thickness of 1 to 100 micrometers, and a formed body having an arbitrary shape cannot be manufactured.

More specifically, only a limited report is found on a monolithic structure that is composed of a polysaccharide or a derivative thereof, and has continuous pores, and from which a formed body having an arbitrary shape can be manufactured, and nothing is known on use of lactic acid as a manufacturing method therefor.

In view of such a situation, an object of the invention is to provide a manufacturing method for a monolithic structure that is a porous body formed of a polysaccharide being a naturally-occurring polymer, has continuous pores having an average pore diameter suitable for biomolecule separation, and allows formation into an arbitrary shape.

### Solution to Problem

The present inventors have diligently continued to conduct research for solving the problem described above, and as a result, have found that a monolithic structure can be easily manufactured from a polysaccharide by selecting a suitable solvent, and thus have completed the invention based on the finding.

More specifically, the invention is as described below.
Item 1. A manufacturing method for a polysaccharide monolithic structure, including: a first step of obtaining a polysaccharide solution by dissolving a polysaccharide into a mixed solvent of a first component and a second component at a temperature lower than a boiling point of the mixed solvent, and a second step of obtaining the polysaccharide monolithic structure by cooling the polysaccharide solution, wherein the first component is a solvent selected from lactate, and the second component is a solvent selected from water, a lower alcohol, and a combination thereof.
Item 2. The manufacturing method according to item 1, wherein the polysaccharide is cellulose.
Item 3. The manufacturing method according to item 1 or 2, wherein at least one hydroxyl group of the polysaccharide is esterified.
Item 4. The manufacturing method according to any one of items 1 to 3, wherein the mixed solvent further contains a third component.
Item 5. The manufacturing method according to item 4, wherein a concentration of the third component in the mixed solvent is 0.1 to 5% by weight.
Item 6. The manufacturing method according to item 4 or 5, wherein the third component is polyethylene glycol.
Item 7. The manufacturing method according to any one of items 1 to 6, wherein a volume ratio of the first component to the second component is 5:95 to 60:40.
Item 8. The manufacturing method according to any one of items 1 to 7, wherein a concentration of the polysaccharide in the mixed solvent is 0.1 to 30% by weight.
Item 9. The manufacturing method according to any one of items 1 to 8, wherein the second step is applied in which cooling is made to a temperature lower by 5 to 200°C than a temperature of the polysaccharide solution before cooling.
Item 10. A manufacturing method for a saponified polysaccharide monolithic structure, including execution of a third step of saponifying the polysaccharide monolithic structure obtained, after the step of manufacturing the polysaccharide monolithic structure by the manufacturing method according to any one of items 1 to 9.
Item 11. A manufacturing method for a cross-linked and/or ligand-introduced polysaccharide monolithic structure, including execution of a fourth step of applying cross-linking and/or ligand-introducing treatment to the saponified polysaccharide monolithic structure obtained, after the step of manufacturing the saponified polysaccharide monolithic structure by the manufacturing method according to item 10.

### Advantageous Effects of Invention

The invention can provide a monolithic structure having a pore diameter suitable for biopolymer separation from an inexpensive polysaccharide or polysaccharide derivative. Moreover, the invention can arbitrarily provide the monolithic structure with adsorption performance to the biopolymer by passing through a step of saponification and cross-linking and/or introduction of ligand, and further can provide a material useful for a method for purifying an objective substance using the monolithic structure.

### Brief Description of Drawings

FIG. 1 is a SEM photograph of a cross section of a monolithic structure in Manufacture Example 3.
FIG. 2 is a SEM photograph of a cross section of a monolithic structure in Manufacture Example 4.
FIG. 3 is a SEM photograph of a cross section of a monolithic structure in Manufacture Example 6.
FIG. 4 is a SEM photograph of a cross section of a monolithic structure in Manufacture Example 7.
FIG. 5 is a SEM photograph of a cross section of a monolithic structure in Manufacture Example 8.
FIG. 6 is a SEM photograph of a cross section of a monolithic structure in Manufacture Example 9.
FIG. 7 is a SEM photograph of a cross section of a monolithic structure in Manufacture Example 11.
FIG. 8 is a SEM photograph of a cross section of a monolithic structure in Manufacture Example 15.

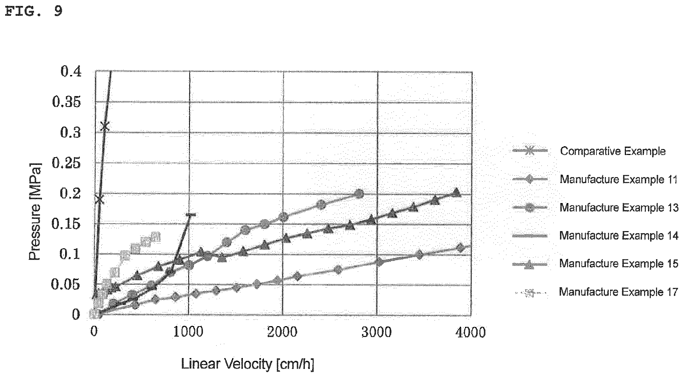

FIG. 9 is a graph showing a relationship between a flow rate and pressure during liquid permeation in Test Example 2.

### Description of Embodiments

A manufacturing method for a polysaccharide structure according to the invention includes a first step of obtaining a polysaccharide solution by dissolving a polysaccharide into a mixed solvent of a first component and a second component at a temperature lower than a boiling point of the mixed solvent, and a second step of obtaining a polysaccharide monolithic structure by cooling the polysaccharide solution.

The polysaccharide used as a material includes a polysaccharide derivative, and specific examples of a preferred material include a polysaccharide a hydroxyl group of which is esterified. When a monolithic structure obtained by using such a polysaccharide derivative is applied as a chromatography base material, nonspecific adsorption can be suppressed. Examples of the esterified polysaccharide include esterified cellulose and esterified glucomannan, and specific examples preferably include cellulose acetate, cellulose propionate, cellulose butyrate, cellulose acetate propionate, cellulose acetate butyrate and glucomannan acetate, and cellulose acetate is particularly preferred thereamong.

A molecular weight of the polysaccharides used as the material is not particularly limited, but in a case of cellulose acetate, for example, an average molecular weight (Mw) is preferably about 1,000 to about 200,000, and further preferably, about 5,000 to about 100,000 from viewpoints of handling properties in the manufacturing process and strength of a formed monolithic structure or a pore diameter of holes of the monolithic structure. In addition, cellulose acetate having an average molecular weight of about 40,000 is available from Wako Pure Chemical Industries, Ltd., and can be preferably used.

The monolithic structure manufactured according to the invention can serve as a column material that can further withstand alkali washing, in comparison with silica being a conventional column material, by adopting the polysaccharide, particularly cellulose as a constitutional material of the monolithic structure. Moreover, the polysaccharide has small nonspecific adsorption, and therefore is suitable as the chromatography base material. Moreover, the polysaccharide has usability as the chromatography base material also in view of the polysaccharide having many hydroxyl groups and ease of introduction of a functional group or a ligand thereinto.

In addition, the functional group or the ligand can also be introduced into a hydroxyl group in silica, but an introduction procedure is complicated, and therefore the introduction is not so practical.

The first component in the manufacturing method according to the invention is a solvent selected from the group of lactate. As the lactate, an ester of lactic acid and a lower alcohol (alcohol having 1 to 5 carbons) is preferred, and methyl lactate or ethyl lactate is further preferred, for example.

The lactate is a safe solvent that is not applicable to regulations by PRTR in the Act on Confirmation, etc. of Release Amounts of Specific Chemical Substances in the Environment and Promotion of Improvements to the Management Thereof, Ordinance on Prevention of Organic Solvent Poisoning, and Ordinance on Prevention of Hazards due to Specified Chemical Substances, and therefore has a load onto workers and an environment is small. Moreover, the lactate is inexpensive. Further, the lactate has a high boiling point, and can efficiently dissolve the polysaccharide by heating. Moreover, the lactate is arbitrarily mixed with water, and therefore water can be used as a cleaning liquid upon removing the solvent from the polysaccharide monolith obtained, and therefore the lactate is economical.

The second component in the manufacturing method according to the invention is a solvent selected from water, a lower alcohol (alcohol having 1 to 5 carbons) and a combination thereof. Examples of the lower alcohol preferably include alkyl alcohol and glycol. The alkyl alcohol is not particularly limited, and specific examples preferably include ethanol and 1-propanol. The glycol is not particularly limited, and specific examples preferably include ethylene glycol, 1,2-propylene glycol and 1,3-butylene glycol.

The second component alone is ordinarily hard to dissolve the polysaccharide hardly at room temperature and cannot dissolve the lactate in an amount of about 0.0001% by weight or more, for example.

In the manufacturing method according to the invention, the polysaccharide is dissolved in the mixed solvent at a temperature lower than the boiling point thereof into the polysaccharide solution in the first step, and the polysaccharide solution is cooled in the second step. The polysaccharide monolithic structure is precipitated and obtained from the polysaccharide solution by phase separation utilizing a temperature difference through the steps. In order to easily realize such phase separation, the mixed solvent of the first component and the second component having different properties as described above is adopted.

With regard to a mixing proportion of the first component to the second component, a proportion of the first component is preferably about 5 to about 60 v/v% and a proportion of the second component is preferably about 95 to about 40 v/v%. If the proportion of the first component is within the above range, precipitation by phase separation is easily caused when the polysaccharide solution is cooled. If the proportion of the second solution is within the above range, the polysaccharide can be easily uniformly dissolved therein.

A proportion of the polysaccharide in the mixed solvent is preferably about 0.1 to about 30% by weight, and further preferably about 1 to about 20% by weight. If the proportion is within the above range, a uniform solution is obtained and a porous body having well-aligned continuous pores is easily obtained. Moreover, when the polysaccharide solution is cooled, precipitation by phase separation is easily caused, and strength of the monolithic structure obtained becomes sufficient and a shape can be easily maintained.

Moreover, as the proportion of the polysaccharide in the mixed solvent is larger, a pore diameter of the continuous pores each of the porous body obtained tends to be smaller. Therefore, when the porous body is applied to chromatography described later, the proportion of the polysaccharide in the mixed solvent is preferably to be adjusted to 5 to 30% by weight, and further preferably, 5 to 20% from a viewpoint of forming the pore diameter with which satisfactory liquid permeability is obtained.

In the manufacturing method according to the invention, the mixed solvent further contains a third component, thereby allowing an increase in the pore diameter of the continuous pores each of the porous body obtained.

As the third component, as long as the third component is soluble in the mixed solvent, any component that is soluble in either the first component or the second component may be satisfactorily applied.

Specific examples of the third component preferably include a component such as a water-soluble polymer including polyethylene glycol (PEG) and polyvinyl alcohol (PVA), a saccharide including glucose, sucrose and starch, and salts including calcium chloride and calcium carbonate, and polyethylene glycol is further preferred.

When the third component is polyethylene glycol, a molecular weight thereof is not particularly limited, but an average molecular weight (Mw) is preferably 100 to 30,000, and further preferably, 1,000 to 10,000, and still further preferably 2,000 to 5,000, from viewpoints of handling properties in the manufacturing process, strength of a formed monolithic structure or a pore diameter of holes of the monolithic structure. In addition, polyethylene glycol having an average molecular weight of 3, 000 is available from Wako Pure Chemical Industries, Ltd., and can be preferably used.

In the second step in the manufacturing method according to the invention, the polysaccharide solution is cooled to a temperature lower by about 5 to about 200°C, preferably, to a temperature lower by about 5°C to about 150°C, and further preferably, to a temperature lower by about 5 to about 100°C, than a temperature before cooling (temperature during the dissolution in the first step). A temperature dropping rate on the above occasion is preferably about 0.1 to about 200°C per minute, and further preferably, about 0.1 to about 100°C per minute.

As described above, the polysaccharide monolithic structure is obtained through the first step and the second step, but treatment described below may be further applied, when necessary, after the second step.

The monolithic structure after the second step includes the mixed solvent used in the first step inside thereof. Therefore, the mixed solvent may be replaced by an arbitrary solvent. A method of replacement is not particularly limited, but the monolithic structure after the second step is immersed into any place in another vessel having an arbitrary solvent therein, thereby allowing replacement by mutual diffusion, for example. Here, the solvent for replacement is desirably miscible with the solvent used in the first step at an arbitrary proportion in order to keep uniformity of the monolithic structure obtained in the second step. Specific examples of a preferred solvent include alcohols and water.

Moreover, treatment for removing the solvent included inside the monolithic structure may be applied thereto. An ordinary method such as heating and pressure reduction can be applied as the method for removal.

Moreover, when the esterified polysaccharide is used as a material in the first step, saponification (de-esterification) may be applied as a third step after the second step. For example, when cellulose acetate is used as a polysaccharide of a material, deacetylation may be applied in the third step. The saponification treatment can be arbitrarily applied according to an ordinary method such as alkali treatment.

Further, a fourth step of introducing an arbitrary functional group or a ligand into a free hydroxyl group thereof may be applied to the saponified (de-esterified) monolithic structure after the third step. Thus, a monolithic structure provided with adsorption performance to an objective substance such as a biopolymer as described above can be obtained and preferably utilized for purification and detection of an objective substance to be described later

The ligand is not particularly limited, and specific examples include an ion exchange group such as 2-diethylaminoethyl (DEAE), carboxymethyl (CM), sulfone, a quaternary ammonium (Q) and sulfate, a hydrophobic group such as a phenyl group and a butyl group, a ligand that can be used for separation of so-called mixed mode having both an ion exchange group and a hydrophobic group, a ligand for protein adsorption such as protein A and an antibody, and a functional polymer such as a polycation including polylysine and a polyanion including heparin and polyglutamic acid, and can be arbitrarily selected according to a purpose of using the monolithic structure, and introduction thereof can be arbitrarily applied according to an ordinary method.

Moreover, as the fourth step, treatment for cross-linking the polysaccharide may be applied in addition to or in place of the introduction of the functional group or the ligand. Strength of the monolithic structure can be improved by providing cross-linking between saccharide chains.

Cross-linking treatment can be arbitrarily applied using a reactive bifunctional reagent as a cross-linking agent according to an ordinary method. Specific examples of the reactive bifunctional reagent preferably include epichlorohydrin, epibromohydrin, diisocyanate, dimethylurea, dimethylethyleneurea, dimethylchlorosilane, bis(2-hydroxyethylsulfone), butanediol diglycidyl ether, ethylene glycol diglycidyl ether, glycerol diglycidyl ether, polyethylene glycol diglycidyl ether, divinylsulfone, alkylene dihalogen and hydroxy alkylene dihalogen, and among the compounds, epichlorohydrin can be particularly preferably used.

The monolithic structure obtained by the manufacturing method according to the invention is a thick stereoscopic body that including the polysaccharide as a main component, and having the continuous pores. In general, monolithic structure means a single block of porous body structure each continuously having three-dimensional network structure skeletons and voids.

Specifically, the monolithic structure obtained by the manufacturing method according to the invention has continuous pores having an average pore diameter of about 0.01 to about 20.0 micrometers. The average pore diameter can be determined from an image photographed using a scanning electron microscope (SEM).

Here, the continuous pores mean continuous voids, and not individually independent cavities. The pores being continuous can be confirmed from a fact that pore shapes are identical or similar in SEM photographs of a plurality of monolithic structure samples, and that column pressure when liquid permeates does not rise easily.

With regard to the shape of pores, the pores on a structure surface and/or inside the structure are preferably circular or elliptical or approximate thereto, but are not particularly limited.

The monolithic structure obtained by the manufacturing method according to the invention has such continuous pores, thereby being excellent in liquid permeability. Moreover, an arbitrary amount of a functional group or a ligand can be introduced thereinto, and reaction efficiency in the functional group or the ligand is satisfactory. Therefore, the monolithic structure is suitable for a column material used for a purification method or the like as described later.

The monolithic structure obtained by the manufacturing method according to the invention has a thickness of about 100 micrometers or more, and preferably, about 150 micrometers or more, and has a stereoscopic form different from a membrane. A shape of the porous body is not limited, but length in a shortest axis direction is referred to as thickness, for convenience, among three directions of depth, width and height of the porous body. Moreover, the monolithic structure obtained by the manufacturing method according to the invention can be formed into an arbitrary shape such as a columnar shape and a cylindrical shape.

The monolithic structure obtained by the manufacturing method according to the invention can be used for chromatography in a form of a column or the like.

The monolithic structure obtained by the manufacturing method according to the invention has continuous pores, and therefore pressure in the column is hard to increase during liquid permeation. Therefore, the monolithic structure allows maintenance or increase of a flow rate, and accordingly is suitable for execution of chromatography.

Moreover, as described above, a desired functional group or ligand can be introduced into the monolithic structure obtained by the manufacturing method according to the invention, and therefore the structure allows provision of adsorption performance to a biopolymer. Various objective substances can be purified using the performance. For example, a monolithic structure into which the ion exchange group is introduced can be used for purifying expression protein, a monolithic structure in which protein A is immobilized as a ligand can be applied to IgG purification, or a monolithic structure in which an antibody is immobilized can be applied to detection or purification of an antigen corresponding to the antibody.

### EXAMPLES

In the following, the invention will be described by way of Examples, but the invention is not limited thereto.

### Manufacture Example 1

### (First step)

In a transparent glass test tube having an inner diameter of 14.0 mm, 500 mg (10% by weight) of cellulose acetate (acetylation degree: 53 to 56%, MW: 40,000, made by Wako Pure Chemical Industries, Ltd.) as a polysaccharide, 1.5 mL (30 v/v%) of ethyl lactate as a first component, 3.0 mL (60 v/v%) of ethanol as a second component and 0.5 mL (10 v/v%) of water were placed, and the resulting mixture was heated by a block heater at 75°C to obtain a polysaccharide solution.

### (Second step)

The polysaccharide solution obtained was naturally cooled to 20 °C in a hot-water bath to obtain a monolithic structure containing a solvent inside pores. The monolithic structure obtained was charged into an excess amount of water in another vessel to obtain a columnar monolithic structure having a diameter of 13.7 mm and containing water in pores.

### Manufacture Example 2

A columnar monolithic structure was obtained in a manner similar to Manufacture Example 1 except that 100 mg of polyethylene glycol 6, 000 (average molecular weight: 7,300 to 9,300, made by Wako Pure Chemical Industries, Ltd.) was simultaneously added thereto as a third component with 500 mg (10% by weight) of cellulose acetate as a polysaccharide.

### Manufacture Example 3

### (First step)

In a transparent glass test tube having an inner diameter of 14.0 mm, 500 mg (10% by weight) of cellulose acetate as a polysaccharide, 2. 0 mL (40 v/v%) of ethyl lactate as a first component, 2.5 mL (50 v/v%) of 1-propanol as a second component and 0.5 mL (10 v/v%) of water were placed, and the resulting mixture was heated by a block heater at 95°C for 3 hours to obtain a polysaccharide solution.

### (Second step)

The test tube containing the polysaccharide solution obtained was transferred to a hot-water bath at 75°C, and cooled to 20°C at a cooling rate of -10°C/h to obtain a columnar monolithic structure containing a solvent inside pores. The monolithic structure obtained was charged into an excess amount of water in another vessel to obtain a columnar monolithic structure containing water inside pores.

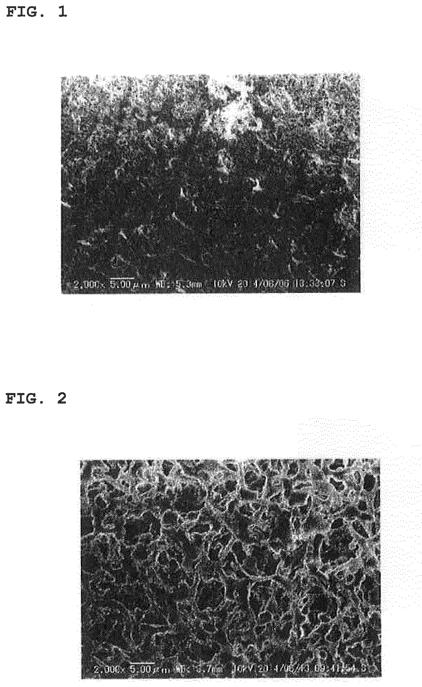

The monolithic structure was cut by an ultrasonic cutter (SONOFILE, SF-30, made by SONOTEC Co., Ltd.), and then frozen at -78 °C, and then dried under reduced pressure at room temperature to obtain a dried monolithic structure. A cross section of the resulting monolithic structure was observed by a scanning electron microscope (SEM) (VE-8800, made by Keyence Corporation), and a photograph obtained is shown in FIG. 1.

### Manufacture Example 4

A columnar monolithic structure was obtained in a manner similar to Manufacture Example 3 except that 190 mg of polyethylene glycol 4, 000 (average molecular weight: 3, 000, made by Wako Pure Chemical Industries, Ltd.) was simultaneously added thereto as a third component with 500 mg (10% by weight) of cellulose acetate as a polysaccharide. FIG. 2 shows a SEM photograph of a cross section of the monolith structure.

### Manufacture Example 5

A columnar monolithic structure was obtained in a manner similar to Manufacture Example 3 except that 50 mg of polyethylene glycol 6,000 was simultaneously added thereto as a third component with 500 mg (10% by weight) of cellulose acetate as a polysaccharide.

### Manufacture Example 6

### (First step)

In a 4 mL glass vial with screw cap, 400 mg (10% by weight) of cellulose acetate as a polysaccharide and 2.48 mL (62 v/v%) of ethyl lactate as a first component were placed, and the resulting mixture was heated and dissolved by a block heater at 100°C. Then, 0.893 g of ethylene glycol (density = 1.114 g/mL) (20 v/v%) was added thereto as a second component, the resulting mixture was heated and dissolved by the block heater at 100°C, and then 0.800 g (18 v/v%) of ethylene glycol was further added thereto as a second component, and the resulting mixture was heated to obtain a polysaccharide solution.

### (Second step)

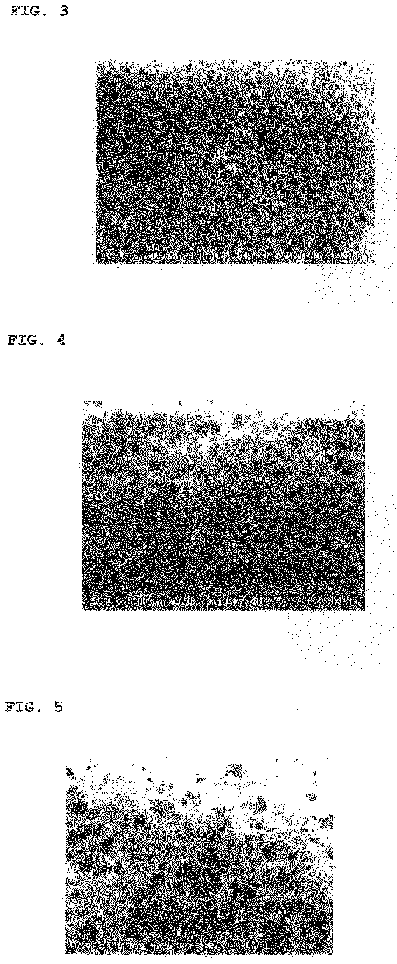

An aluminum block heater was cooled from 75°C to 20°C at a cooling rate of -10°C/h to obtain a monolithic structure containing a solvent inside pores. The monolithic structure obtained was charged into an excess amount of water in another vessel to obtain a columnar monolithic structure containing water inside pores. FIG. 3 shows a SEM photograph of a cross section of the monolithic structure.

### Manufacture Example 7

### (First step)

In a 4 mL glass vial with screw cap, 400 mg (10% by weight) of cellulose acetate as a polysaccharide and 2.48 mL (62 v/v%) of ethyl lactate as a first component were placed, and the resulting mixture was heated by a block heater at 100°C. Then, 0.800 g of ethylene glycol (density = 1.114 g/mL) (18 v/v%) was added thereto as a second component and the resulting mixture was heated and dissolved at 100°C, and then 0.500 g (10 v/v%) of an ethylene glycol solution of 10% by weight of polyethylene glycol 20,000 (average molecular weight 20,000 ± 5,000, made by Wako Pure Chemical Industries, Ltd.,) as a third component and 0.433 g (10 v/v%) of ethylene glycol as a second component were further added thereto, and the resulting mixture was heated to obtain a polysaccharide solution.

### (Second step)

An aluminum block heater was cooled from 75 °C to 20 °C at a cooling rate of -10 °C/h to obtain a monolithic structure containing a solvent inside pores. The monolithic structure obtained was charged into an excess amount of water in another vessel to obtain a columnar monolithic structure containing water inside pores. FIG. 4 shows a SEM photograph of a cross section of the monolithic structure.

### Manufacture Example 8

### (First step)

In a 4 mL glass vial with screw cap, 400 mg (10% by weight) of cellulose acetate as a polysaccharide, 2.27 g of ethyl lactate (density = 1.034 g/mL) (55 v/v%) as a first component and 0.83 g of 1,2-propylene glycol (density = 1. 036 g/mL) (20 v/v%) as a second component were placed, and the resulting mixture was heated and dissolved by a block heater at 100°C. Further, 1.04 g (25 v/v%) of 1, 2-propylene glycol was added thereto as a second component, and the resulting mixture was heated at 100°C to obtain a polysaccharide solution.

### (Second step)

An aluminum block heater was cooled from 75°C to 20°C at a cooling rate of -10 °C/h to obtain a monolithic structure containing a solvent inside pores. The monolithic structure obtained was charged into an excess amount of water in another vessel to obtain a columnar monolithic structure containing water inside pores. FIG. 5 shows a SEM photograph of a cross section of the monolithic structure.

### Manufacture Example 9

### (First step)

In a 4 mL glass vial with screw cap, 400 mg (10% by weight) of cellulose acetate as a polysaccharide 2.688 mL (65 v/v%) of ethyl lactate as a first component and 0.402 g of 1,3-buthylene glycol (density = 1.005 g/mL) (10 v/v%) as a second component were placed, and the resulting mixture was heated and dissolved by a block heater at 100°C. Further, 1.005 g (25 v/v%) of 1,3-butylene glycol was added thereto, and the resulting mixture was heated at 100°C to obtain a polysaccharide solution.

### (Second step)

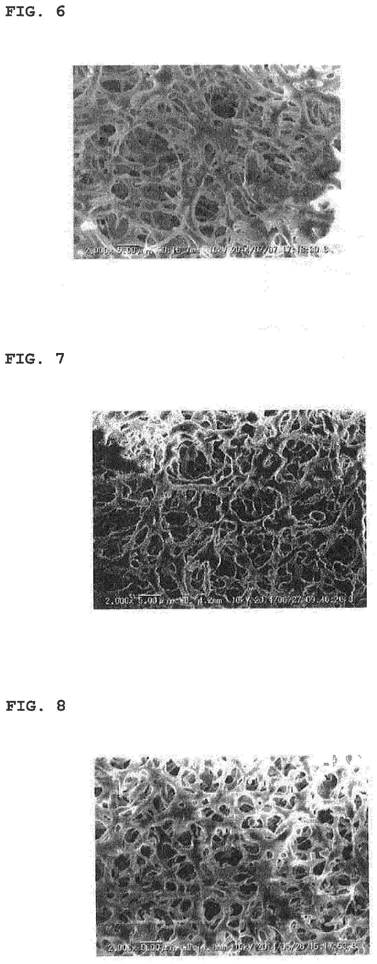

An aluminum block heater was cooled from 75°C to 20°C at a cooling rate of -10°C/h to obtain a monolithic structure containing a solvent inside pores. The monolithic structure obtained was charged into an excess amount of water in another vessel to obtain a columnar monolithic structure containing water inside pores. FIG. 6 shows a SEM photograph of a cross section of the monolithic structure.

### Manufacture Example 10

A columnar monolithic structure was obtained in a manner similar to Manufacture Example 3 except that a composition of a mixed solvent was changed to a mixture of 1.5 mL (30 v/v%) of methyl lactate as a first component, 3.0 mL (60 v/v%) of 1-propanol as a second component and 0.5 mL (10v/v%) of water.

### Manufacture Example 11

### (First step)

In a 300 mL recovery flask, 60.0 mL (40 v/v%) of ethyl lactate as a first component, 35.0 mL (23.3 v/v%) of 1-propanol as a second component and 15.0 mL (10v/v%) of water were placed, the resulting mixture was mixed, and 15.0 g (10% by weight) of cellulose acetate was added thereto as a polysaccharide, and the resulting mixture was heated and dissolved by a block heater at 95°C for 3 hours. A temperature of the block heater was reduced to 75°C, 40.0 mL (26.7 v/v%) of 1-propanol as a second component and 5.7 g of polyethylene glycol 4,000 as a third component were added thereto, and the resulting mixture was heated by the block heater at 95°C for 1 hour to obtain a polysaccharide solution.

### (Second step)

The temperature of the block heater was reduced to 75°C, and the polysaccharide solution was dispensed to a glass test tube having an inner diameter of 14.0 mm and preliminarily heated to 75°C by the block heater. The glass test tube containing the polysaccharide solution obtained was transferred to a hot-water bath at 75°C and cooled to 20 °C at a cooling rate of -10°C/h to obtain a monolithic structure containing a solvent inside pores. The monolithic structure obtained was charged into an excess amount of water in another vessel to obtain a columnar monolithic structure containing water inside pores. FIG. 7 shows a SEM photograph of a cross section of the monolithic structure.

### Manufacture Example 12

### (Third step)

In a test tube having an inner diameter of 15.4 mm, 1 mL of a 10 M sodium hydroxide aqueous solution and 1 mL of water were placed, the resulting solution was well stirred, a piece of the monolith that was obtained in Manufacture Example 11 and cut into a length of 1 cm by an ultrasonic cutter was added thereto, and the resulting mixture was shaken at 30°C for 5 hours to achieve deacetylation. The deacetylated monolith obtained was charged into an excess amount of water in another vessel to remove the alkali aqueous solution contained inside the pores of the monolithic structure.

### (Fourth step: cross-linking)

Next, 0.35 mL of epichlorohydrin and 5.0 mL of water were added into the test tube to dissolve epichlorohydrin thereinto by using a vortex generator. A piece of deacetylated monolith was added to the test tube containing the epichlorohydrin aqueous solution, and the resulting mixture was shaken at 30°C for 1 hour. Further, 0.45 mL of a 10 M sodium hydroxide aqueous solution and 2.55 mL of water were added into another test tube, and the resulting mixture was well stirred, and a piece of monolith structure into which the epichlorohydrin aqueous solution was immersed was added thereto, and the resulting mixture was shaken at 50°C for 2 hours to perform a cross-linking reaction. The immersion of the epichlorohydrin aqueous solution and the reaction by the sodium hydroxide aqueous solution were further repeated once to perform the cross-linking reaction two times in total. After reaction completion, the monolith structure was charged into an excess amount of water in another vessel to replace the solvent contained inside the monolith structure pores by water.

### Manufacture Example 13

### (Fourth step: introduction of ligand)

In a test tube having an inner diameter of 15.4 mm, 1.4 g of 2-diethylaminoethyl chloride hydrochloride and 2.0 mL of water were placed, the resulting solution was well stirred, a piece of the monolith obtained in Manufacture Example 12 was added thereto, and the resulting mixture was shaken at 30°C for 1 hour. Further, in another test tube, 1.7 mL of a 10 M sodium hydroxide aqueous solution and 1.3 mL of water were placed, the resulting solution was well stirred, a monolith structure into which a 2-diethylaminoethyl chloride hydrochloride was immersed was added thereto, and the resulting mixture was shaken at 50°C for 2 hours to perform a reaction of introducing diethylaminoethyl. The immersion of the 2-diethylaminoethyl chloride hydrochloride aqueous solution and the reaction by the sodium hydroxide aqueous solution were further repeated once to perform the reaction of introducing DEAE two times in total. After reaction completion, the monolith structure was charged into an excess amount of water in another vessel to replace the solvent contained inside the monolith structure pores by water.

### Manufacture Example 14

### (Fourth step: introduction of ligand)

In a test tube having an inner diameter of 15.4 mm, 1.69 g (73.2% by weight) of glycidyltrimethylammonium chloride aqueous solution and 1.55 mL of water were placed, the resulting solution was well stirred, a piece of the monolith structure in Manufacture Example 12 was added thereto, and the resulting mixture was shaken at 30°C for 1 hour. Further, in another test tube, 1.7 mL of a 10 M sodium hydroxide aqueous solution and 1.3 mL of water were placed, the resulting mixture was well stirred, a monolith into which the glycidyltrimethylammonium chloride aqueous solution was immersed was added thereto and the resulting mixture was shaken at 50°C for 2 hours to perform a reaction of introducing trimethylammonium. The immersion of the glycidyltrimethylammonium chloride aqueous solution and the reaction by the sodium hydroxide aqueous solution were further repeated once to perform the reaction of introducing trimethylammonium two times in total. After reaction completion, the monolith structure was charged into an excess amount of water in another vessel to replace the solvent contained inside the monolith structure pores by water.

### Manufacture Example 15

### (First step)

In a 100 mL recovery flask, 5.0 g (10% by weight) of cellulose acetate as a polysaccharide and 9.5 mL (19 v/v%) of ethylene glycol as a second component were placed, and the resulting mixture was stirred. While continuing stirring in the recovery flask, 31.0 mL (62 v/v%) of ethyl lactate as a first component was added thereto, and the resulting mixture was heated and dissolved by a hot-water bath at 95°C for 4 hours. A temperature of the hot-water bath was reduced to 75°C, 0.625 g of polyethylene glycol 20,000 as a third component and 9.5 mL (19 v/v%) of ethylene glycol as a second component were added thereto, and the resulting mixture was heated by a hot-water bath at 95°C for 1 hour to obtain a polysaccharide solution.

### (Second step)

The temperature of the hot-water bath was reduced to 75°C, and a polysaccharide solution was dispensed to a test tube preliminarily heated at 75°C by a hot-water bath. The test tube containing the polysaccharide solution obtained was transferred to a hot-water bath at 75°C and cooled to 20°C at a cooling rate of -10°C/h to obtain a monolithic structure containing a solvent inside pores. The monolithic structure obtained was charged into an excess amount of water in another vessel to obtain a columnar monolithic structure containing water inside pores. FIG. 8 shows a SEM photograph of a cross section of the monolithic structure.

### Manufacture Example 16

### (Third step) (Fourth step: cross-linking)

A cross-linked monolithic structure was obtained in a manner similar to Manufacture Example 12 except that the monolithic structure in Manufacture Example 15 was used.

### Manufacture Example 17

### (Third step) (Fourth step: cross-linking and introduction of ligand)

A DEAE-introduced monolithic structure was obtained in a manner similar to Manufacture Example 13 except that the monolithic structure in Manufacture Example 16 was used.

### Test Example 1

### Evaluation of hardness

The water-containing columnar monolithic structures obtained in Manufacture Examples 3, 4, 5, 6, 7, 8, 9, 11 and 15 were cut by an ultrasonic cutter into a length of 0.5 to 1.0 cm. Next, hardness was measured by pressing a durometer (GS-743G, made by TECLOCK Corporation) against a cross section of the cut monolith structure. Table 1 shows the results.
**Table 1**

**Table 1**

| | Hardness |
|---|---|
| Manufacture Example 3 | 78 |
| Manufacture Example 4 | 60 |
| Manufacture Example 5 | 73 |
| Manufacture Example 6 | 66 |
| Manufacture Example 7 | 53 |
| Manufacture Example 8 | 48 |
| Manufacture Example 9 | 63 |
| Manufacture Example 11 | 61 |
| Manufacture Example 15 | 74 |

### Test Example 2

### Evaluation based on flow rate and pressure during liquid permeation

Liquid permeation was evaluated by allowing pure water to permeate through the monolithic structures obtained in Manufacture Examples 11, 13, 14, 15 and 17, and measuring pressure applied to a column inlet. Column manufacture procedures are as described below.

The columnar monolithic structure obtained was cut into a length of 1.0 cm by using an ultrasonic cutter. Both ends of the monolithic structure were interposed between a 5.0 cm tetrafluoride pipe (I.D. 7 mm, O.D. 9 mm) and a polystyrene frit having a diameter of 9.3 mm and a thickness of 2.4 mm, and the resulting set was placed in a piece of Sumitube C (SUMI-C-14, made by Sumitomo Electric Fine Polymer, Inc.) having a length with which a full length of the set was covered, and the whole set was heated in a hot-water bath at 95°C to cause shrinkage of the Sumitube. The resulting structure covered with the shrunk Sumitube was placed in a Teflon (registered trademark) heat-shrinkable tube (FEP-120, purchased from AS ONE Corporation) cut into a length of 5 cm in such a manner that the monolith part was placed in a center of the heat-shrinkable tube, and the whole assembly was heated by a heat gun to cause shrinkage, thereby producing a column. Profile unions (U-20C, made by Tokyo Rikakikai Co., Ltd.) were connected to both ends of the resulting column device including the monolithic structure. A tube having an outer diameter of 6 mm was connected to allow liquid permeation.

In evaluating liquid permeability, a pump (LC8A, made by Shimadzu Corporation) and a pressure gauge (GP-M025, made by Keyence Corporation) were used, and a value indicated on the pump was used for flow rate evaluation. Further, a flow rate per unit area was calculated, and the liquid permeability of various monolithic structures was compared by adopting the calculated value as linear velocity.

As Comparative Example, a column packed with spherical particles (Cellufine A-500, 5 mL Mini-Column, made by JNC CORPORATION) was used, and an evaluation was made in a similar manner.

FIG. 10 shows the results. The monolithic structures obtained by the manufacturing method according to the invention were found to allow liquid permeation at a low pressure and a high flow rate.

### Test Example 3

### Evaluation by adsorptive properties of bovine serum albumin (BSA)

BSA adsorptive properties were measured by permeating a BSA aqueous solution into a column that employs diethylaminoethyl-introduced or trimethyl ammonium-introduced monolith structure obtained in Manufacture Examples 13, 14 and 17. Procedures for column preparation and adsorptive property evaluation are as described below.

Both ends of the monolith were interposed between a piece of silicone rubber penetrated by a PEEK tube and a polypropylene frit, the resulting set was further placed in a piece of Sumitube C having a length with which a full length of the set was covered, and the whole set was heated in a 95°C hot-water bath to cause shrinkage of the Sumitube. Further, the resulting set was placed in a Teflon (registered trademark) heat-shrinkable tube (FEP-120) with which a full length of the set was covered, and the whole assembly was heated by a heat gun to cause shrinkage, thereby producing a column.

The column thus produced was installed into a low pressure chromatography system (AKTAprime plus, made by GE Healthcare). A BSA solution adjusted to a concentration of 1 mg/mL (50 mM tris(hydroxymethyl)aminomethane hydrochloride, pH 8.3) was allowed to permeate through the chromatography system at a flow rate of 3 mL/min to allow the BSA to adsorb on the column. Moreover, an eluate (1 M sodium chloride, 50 mM tris (hydroxymethyl) aminomethane hydrochloride, pH 8.3) was allowed to permeate to elute BSA from the column. BSA adsorptive properties were measured by determining an amount of BSA contained in the eluate from the column. With regard to dynamic binding capacity (DBC), an amount of adsorption at a moment when 10% of the permeated BSA solution has passed was evaluated as 10% DBC. Static binding capacity (SBC) was evaluated using an amount of elution from the column. Table 2 shows results of DBC and SBC.
**Table 2**

**Table 2**

| | 10% DBC (mg/mL) | SBC (mg/mL) |
|---|---|---|
| Manufacture Example 13 | 22 | 32 |
| Manufacture Example 14 | 25 | 25 |
| Manufacture Example 17 | 11 | 22 |

### Test Example 4

### Measurement of ion exchange capacity of ligand-introduced monolith

After measuring a diameter and height of the diethylaminoethyl-introduced monolithic structure obtained in Manufacture Example 13 or the trimethylammonium-introduced monolithic structure obtained in Manufacture Example 14, the monolithic structure was pulverized using a mixer (Fiber mixer MX-X103, made by Panasonic Corporation), and the pulverized monolithic structure was collected on Kiriyama funnel filter paper. An excessive amount of 0.5 M NaOH aqueous solution was poured onto the pulverized monolith on a filter paper, and then the monolith was washed with water until the monolith became neutral. The pulverized monolith on the filter paper was collected, and water was added thereto until a total weight including the pulverized monolith reached 8 g. Further, 2 mL of a 0.5 M HCl aqueous solution was added thereto, and the resulting mixture was shaken for 1 hour. A suspension containing the pulverized monolith was left to stand, and 2 mL of supernatant was taken and titrated by a 0.1 M NaOH aqueous solution. A precipitate of the suspension containing the pulverized monolith was collected on a piece of filter paper, dried and weighed. In calculation of ion exchange capacity, a value obtained by subtracting dry weight of the pulverized monolith from 8 g, which is the total of the pulverized monolith and water, was assumed to be an amount of moisture. Table 3 shows the results.
**Table 3**

**Table 3**

| | meq/mL |
|---|---|
| Manufacture Example 13 | 0.33 |
| Manufacture Example 14 | 0.14 |

### Industrial Applicability

The invention can provide a monolithic structure having a pore diameter suitable for biopolymer separation from an inexpensive polysaccharide or a polysaccharide derivative. Moreover, the invention can arbitrarily provide the monolithic structure with adsorption performance to a biopolymer to contribute to improvement of efficiency in a purification step in manufacturing a medicine or the like, and therefore is industrially very useful.

## Claims

1. A manufacturing method for a polysaccharide monolithic structure, comprising:
a first step of obtaining a polysaccharide solution by dissolving a polysaccharide into a mixed solvent of a first component and a second component at a temperature lower than a boiling point of the mixed solvent, and
a second step of obtaining the polysaccharide monolithic structure by cooling the polysaccharide solution, wherein
the first component is a solvent selected from lactates, and
the second component is a solvent selected from water, a lower alcohol, and a combination thereof.

2. The manufacturing method according to claim 1, wherein the polysaccharide is cellulose.

3. The manufacturing method according to claim 1 or 2, wherein at least one hydroxyl group of the polysaccharide is esterified.

4. The manufacturing method according to any one of claims 1 to 3, wherein the mixed solvent further contains a third component.

5. The manufacturing method according to claim 4, wherein a concentration of the third component in the mixed solvent is 0.1 to 5% by weight.

6. The manufacturing method according to claim 4 or 5, wherein the third component is polyethylene glycol.

7. The manufacturing method according to any one of claims 1 to 6, wherein a volume ratio of the first component and the second component is 5:95 to 60:40.

8. The manufacturing method according to any one of claims 1 to 7, wherein a concentration of the polysaccharide in the mixed solvent is 0.1 to 30% by weight.

9. The manufacturing method according to any one of claims 1 to 8, wherein, in the second step, the polysaccharide solution is cooled to a temperature lower by 5 to 200°C than a temperature before cooling.

10. A manufacturing method for a saponified polysaccharide monolithic structure, comprising a third step of saponifying the polysaccharide monolithic structure obtained, after the step of manufacturing the polysaccharide monolithic structure by the manufacturing method according to any one of claims 1 to 9.

11. A manufacturing method for a cross-linked and/or ligand-introduced polysaccharide monolithic structure, comprising a fourth step of applying cross-linking and/or ligand-introducing treatment to the saponified polysaccharide monolithic structure obtained, after the step of manufacturing the saponified polysaccharide monolithic structure by the manufacturing method according to claim 10.
